(19) Europäisches Patentamt
European Patent Office
Office européen des brevets

(11) Publication number: **0 309 299**
**A1**

(12) # EUROPEAN PATENT APPLICATION

(21) Application number: **88308919.5**

(22) Date of filing: **23.09.88**

(51) Int. Cl.[4]: **G 01 N 33/531**
C 07 K 15/00, G 01 N 33/94,
C 12 P 21/00

(30) Priority: **24.09.87 GB 8722470**

(43) Date of publication of application:
**29.03.89 Bulletin 89/13**

(84) Designated Contracting States:
**AT BE CH DE ES FR GB GR IT LI LU NL SE**

(71) Applicant: **CAMBRIDGE LIFE SCIENCES PLC**
**Cambridge Science Park Milton Road**
**Cambridge CB4 4GN (GB)**

(72) Inventor: **Blackmore, David John**
**18a The Crescent Littleport**
**Ely Cambridgeshire (GB)**

**Jackman, Roy**
**13 Wingfield Close New Haw**
**Surrey KT15 3BX (GB)**

**Morris, John Anthony**
**156 McDonald Road**
**Lightwater Surrey GU18 5YB (GB)**

(74) Representative: **Lambert, Hugh Richmond et al**
**D. YOUNG & CO. 10 Staple Inn**
**London, WC1V 7RD (GB)**

(54) **Method for the production of antigenic protein-hapten conjugates, and antibodies corresponding thereto.**

(57) Antigenic protein-hapten conjugates (immunogens) are produced in water-insoluble form from water-labile haptens, such as penicillin and other beta-lactam antibiotics, by reacting the protein and the water-labile hapten in aqueous solution under conditions, e.g. an excess of cross-linking agent, which cause substantially simultaneous precipitation of the protein-hapten conjugate before any substantial hydrolysis of the water-labile hapten has occurred. Such immunogens are used to raise antibodies, e.g. anti-penicillin antibodies, having specificity to the free, non-derivatised hapten, e.g. free non-derivatised penicillin. Such anti-penicillin antibodies are particularly useful in immunoassay of penicillin and other beta-lactam antibodies in milk.

EP 0 309 299 A1

## Description

# METHOD FOR THE PRODUCTION OF ANTIGENIC PROTEIN-HAPTEN CONJUGATES, AND ANTIBODIES CORRESPONDING THERETO

FIELD OF INVENTION

This invention relates to the production of antigenic protein-hapten conjugates (immunogens) of water-labile haptens, and to the production of antibodies having a specificity towards water-labile haptens.

Whilst the invention relates to water-labile haptens in general, and to the production of the corresponding immunogens and antibodies, the invention specifically relates to, and will be described with reference to, the production of penicillin immunogens and anti-penicillin antibodies. However, the techniques taught herein may be applied to the production of other immunogens and antibodies where a similar problem arises, namely that of water-sensitivity of the immunogen or hapten in question, which may be a drug or pharmaceutical such as penicillin, or any other water-sensitive or water-labile compound that it is desired to detect and/or quantify by immunoassay.

BACKGROUND AND PRIOR ART

Anti-penicillin antibodies are known and are commercially available. However, the applicants have found that such antibodies exhibit no or hardly any affinity for free, non-derivatised penicillin, although exhibiting high affinity for the originating immunogen and to protein-penicillin conjugates. The need therefore exists for antibodies having a high affinity for free, non-derivatised penicillin, i.e. "native" penicillin.

As prior art located in the course of preparing this application, there may be mentioned:

GB-A-1,058,828. This discloses the manufacture of artificial antigens which comprises covalently linking a serologically determinant peptide to a carrier protein. The conjugate is then used to raise antibodies specific to the peptide antigen in the normal manner. At page 4 lines 4-10 of the description, it is suggested that an alternative method of isolating the conjugate is by precipitation with a protein precipitant. However, the process does not relate specifically to water labile haptens, nor does it recognise any particular problem associated with the recovery of antigenic protein conjugates of water soluble haptens.

GB 2,160,312 B. This discloses producing a water insoluble immunogen in particulate form, see page 7 lines 7-21, but without specifically addressing the problem solved by the present invention that is to say the formation of immunogens from water-labile haptens but without undue degradation of the hapten during the conjugation process. More particularly, GB 2,160,312 B is directed to an immunising agent or preparation comprising particles of coalesced and covalently interlinked physiologically compatible proteinaceous molecules and, entrapped in these particles, an immunogen adapted to be released by the gradual proteolysis of the particles and, when so released, being of sufficient size to elicit an immuno response to characteristic immunogen determinants of the immunogen, and a process for the manufacture of such an immunising agent or preparation which comprises polycondensing (polymerising by covalent reaction) the proteinaceous molecules in a solution thereof containing also dissolved or suspended an immunogen or hapten, using a difunctional linking agent or other polycondensating agent for the proteinaceous molecules, if required in the presence of an emulisfying agent, and dispersing the resulting suspension with controlled and prolonged agitation in an oil solution to produce an emulsion, followed by coalescence of the polycondensed proteinaceous molecules into beads, and in the case of a hapten being employed, linking such hapten during or prior to the aforesaid process steps sufficiently strongly to a carrier suitable to transform the hapten into an immunogen. The specific object is to provide an immunising agent or preparation, more particularly injectable and preferably for use as a vaccine, comprising features which in combination provide an adjuvant effect and including a prolonged immunogenic stimulus.

EP-A-0 144 154. In contrast to the two foregoing publications, which have no specific reference to the production of penicillin antigen, EP-A-0 144 154 is specifically directed to the preparation of penicillin antigens by the formation of a penicillin protein conjugate. On the other hand, in contrast to the present invention, it specifically directs, at page 5 lines 29 and 30, that the beta-lactam ring of the parent penicillin compound should be hydrolyzed to produce a penicilloic acid conjugate. This therefore goes directly against the teachings of the present invention, the object of which is to avoid hydrolysis of the beta-lactam ring thereby to keep the penicillin intact.

International Application WO 81/02792. Like the previous publication, WO 81/02792 is at least relevant to the background of the present invention in that it is specifically directed to a method for the determination of the presence of penicillin and other lactam containing antibiotics in milk, and using an immobilised antibody to the antibiotic raised from an antigenic protein-hapten, e.g. protein/penicillin, conjugate. However, they appear not even to recognise, let alone deal with, the problem solved by the present invention, namely that the penicillin molecule, and similarly other beta-lactam antibiotic molecules, is water labile so that the method they describe for the production of the penicillin protein conjugate, see page 9 line 20 through to page 10 line 4, will result in substantial degradation of the penicillin molecule. Broadly speaking this method comprises:

(i) covalently conjugating the antibiotic having a lactam-ring in the molecule, e.g. benzyl penicillin, to a protein capable of binding thereto through said lactam-ring;

(ii) injecting into a host animal capable of raising antibodies specific to said antibiotic the conjugate

obtained in step (i) so as to raise said specific antibodies;

(iii) covalently conjugating the same antiboitic as used in step (i) to a second protein capable of binding thereto through said lactam ring and different than the protein used in step (i) to form a second conjugate;

(iv) covalently binding said second conjugate to a solid matrix to form an affinity matrix for purifying the antibodies;

(v) isolating and purifying the specific antibodies raised in step (ii) by contacting the host animal serum with the affinity matrix; and

(vi) recovering the specific antibodies in pure form.

International Publication WO 86/03841. Again, this is of relevance to the background of the present invention. In particular, it specifically recognises the problems of raising antibodies to beta-lactam antibiotics, and in particular the problems caused by opening of the beta-lactam ring during conjugation, see page 5 lines 1-7. Their answer is to use cross-reactive antibodies to measure not only native analyte present in the sample, but also the altered analyte. But when it comes to raising the antibody to the native analyte, e.g. penicillin itself, pages 21-25, they appear to use a method which itself is open to the very problem which they describe, namely opening of the lactam ring during conjugation. Thus, whilst recongising that a problem exists with water labile haptens, they do not appear to have solved it, and certainly not by the method employed by the present invention.

Chem. Abs., Vol. 90, 1979,202024e. This merely describes the production of immunogens from acid labile oligosaccharides by conjugation with a protein. For this purpose the oligosaccharides are reacted with 2-(4-aminophenyl)ethylamine thereby to introduce arylamine groups into the terminal reducing end of the oligosaccharide and which are subsequently converted to phenylisothiocyanate derivatives capable of covalently coupling to free lysylamine groups in the protein.

J. Biochem Vol. 84, 1978, 491-494 (Chem. Abs. Vol. 89, 1978, 173256d). This discloses a three stage method for the formation of penicilln protein conjugates, but without apparently recognising the problems resulting from degradation of the water labile hapten. In this method maleimide groups are first introduced into the penicillin by reaction with N(maleimidobenzoyloxy) succinimide, which are then used to form covalent thioether bonds with cleaved disulfide bonds in the protein (BSA).

Avrameas, J. Biol. Chem. Vol. 242, 1967, 1651-59. This article merely concerns the production of protein antigens in water insoluble form by cross-linking with ethyl chloroformate. Such cross-linked antigens are shown to retain their immunogenic properties. Antigenic penicillin conjugates are not involved.

Chem. Abs., Vol. 103, 1985,214t. Monoclonal antibodies specific to the benzylpenicilloyl group of penicillin are disclosed, but not their method of preparation. The specificity of the monoclonal antibodies to bound penicillin derivatives was measured and shown to be independent of the nature of the side chain.

Chem. Abs. Vol 107, 1987, 89344p. This discloses an anti-benzylpenicilloyl (BPO) monoclonal IgE antibody prepared from the spleens of immune mice whose sera reacted with BPO-hapten penicillin G (PCG) polymer, cephalothin (CET) hapten and CET polymer. It was shown that the haptenic specificity of this antibody was directed mainly to the phenylacetyl portion of the BPO and possessed cross-reactivity with BPO hapten, CET hapten, ampicillin hapten and cefalozin hapten ascribable to the similarity of the antibiotic acyl side chains. Again this has no recognition of the problems of raising antibodies to water-labile haptens using known methods of obtaining the antigenic protein-hapten conjugates.

Finally US-A-4,347,312 discloses a competitive ELISA method for detecting antibiotics in milk, e.g. penicillin, using an immobilised anti-penicillin antibody. Also disclosed is a method of raising the anti-penicillin antibody by injecting a host animal with a penicillin/protein conjugate and separating the anti-penicillin antibody by affinity chromatography using a second penicillin/protein conjugate covalently bonded to a solid matrix, the protein component of the second conjugate being different from that of the first, i.e. the penicillin/protein conjugate used to raise the antibody in the first place. In preparing that conjugate, the penicillin is added to bovine gamma globulin in a sodium carbonate buffer at a temperature of 4°C and adjusted to a pH of 9.6. Further quantities of penicillin were added after 16 and 8 hour intervals. After a further 12 hour period, the mixture is eluted thorugh a Sephadex G-200 column, treated with cysteine hydrochloride and incubated for 1 hour at 37°C. The resulting product was eluted again through a Sephadex G-200 column following which the PEN-BGG conjugate was recovered by lyophilisation. Again there is no recognition that there is a problem in forming antibodies in aqueous solution from water labile haptens such as penicillin.

No prior art therefore confronts the same problem as dealt with by the present invention, namely that in conventional and known methods for the preparation of antigenic protein conjugates of water-labile haptens, no account is taken of the water-labile nature of the hapten, with the consequence that the protein/hapten conjugate produced is that of a hydrolysed hapten derivative, rather than of the native hapten itself, with the further consequence that the resulting antibody raised by that conjugate is specific not to the native hapten, but to a derivatised form of that hapten. In the specific case of beta-lactam antibodies such as penicillin, it is the beta-lactam ring in particular that is water labile or water sensitive, with the result that using conventional or known methods of producing antigenic protein/penicillin conjugates, the resulting antibody is specific to the open chain derivative rather than the original native penicillin containing an intact beta-lactam ring.

Throughout this specification and claims, and as is in common usage, the term"penicillin" is used generically to denote naturally occuring and synthetic derivatives of 6-aminopenicillanic acid (6-APA):

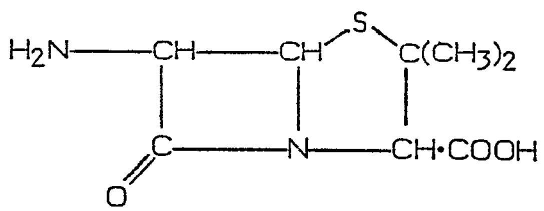

viz : compounds of the formula:

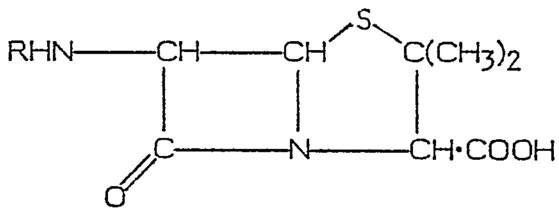

where R is, for example, benzyl (benzyl penicillin; penicillin G), 2-pentenyl (penicillin F), alkylmercaptomethyl (pencillin O), alpha-aminobenzyl (ampicillin) etc., and including 6-APA itself and penicillanic acid, viz: the compound

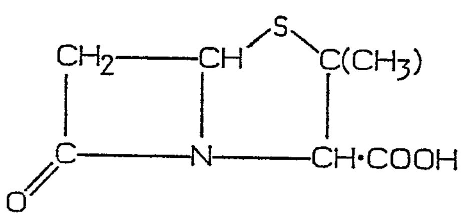

The term "non-derivatised" refers to native penicillin in free form, free from conjugating protein.

SUMMARY OF INVENTION

The present invention is based on the recognition by the inventor of the known fact that the penicillin nucleus, and other similar beta-lactam structures, is water-labile, and ruptures in aqueous media, particularly when in contact with water for any length of time. Recognising this fact, antibodies having high affinity to free, non-derivatised native penicillin have now been produced using penicillin immunogens which are substantially insoluble in water and which are injected into the host animal in a suitable carrier, preferably a non-aqueous injectable carrier. Such water-insoluble immunogens may be obtained, in accordance with a second aspect of the present invention, by reacting the water-labile hapten with a protein in aqueous solution to form a water-soluble antigenic protein-hapten conjugate (immunogen) and precipitating the protein-hapten conjugate from solution before any substantial degree of degradation takes place of the water-labile hapten. Preferably, the protein-hapten conjugate is precipitated substantially instantaneously from solution, for example by using an excess of coupling agent effective not only to couple the hapten to the protein, but also to effect the substantially simultaneous cross-linking of the protein with consequent substantially simultaneous precipitation of the protein-hapten conjugate in water-insoluble form. Other methods of protein insolubilisation may also be used, e.g. salting out, pH adjustment, etc.

In a less preferred alternative, the protein may be insolubilzed prior to conjugation with the hapten, e.g. by pH adjustment, heat treatment, or cross-linking, following which the hapten is conjugated with the insoluble protein to provide a water-insoluble conjugate.

DETAILED DESCRIPTION

In a broad aspect, therefore, the present invention relates to a method of producing an antigenic protein-hapten conjugate from a water-labile hapten, which comprises forming the protein-hapten conjugate in an aqueous medium, and recovering the conjugate in water-insoluble form from the aqueous medium before any substantial degree of degradation has taken place of the water-labile hapten.

In a second aspect, the present invention relates to a method of raising antibodies in a host animal, which antibodies are specific to a water-labile hapten, which comprises injecting into the host animal a

protein-hapten conjugate capable of stimulating the production of said antibodies by the host animal, and recovering the antibodies, or animal tissue cells capable of producing said antibodies, from the host animal, wherein the said protein-hapten conjugate is used in water-insoluble form.

In principle, any method of coupling the hapten to the protein, either soluble or insoluble, may be employed to form the protein-hapten conjugate, but as indicated, the preferred technique is to employ a coupling agent, which when used in excess of that required for coupling, also causes cross-linking and consequent insolubilisation of the protein, thereby precipitating the protein-hapten conjugate out of solution virtually simultaneously. Suitable cross-linking agents for this purpose include traditional and known cross-linking agents such as MBS(I) (maleimidobenzoyl-N-succinimide) MBS(II) (maleimidobutyryloxy-N-succinimide), DMA (dimethyladipimidate), carbodiimide, and isobutylchloroformate.

Where the protein-hapten conjugate is subsequently precipitated from solution, e.g. by pH adjustment or salting out, such subsequent treatment should, of course, be carried out substantially immediately after formation of the conjugate, and before any substantial degradation occurs of the water-labile hapten. Depending upon the actual sensitivity of the hapten to water, the protein-hapten conjugate may be precipitated from solution several minutes after formation, e.g. upto 15 to 20 minutes, but preferably, and more usually, within 2 to 3 minutes.

As already indicated, the invention is particularly directed to penicillin-containing immunogens, i.e. protein-penicillin conjugates. As the penicillin moiety there may be used any of the specific penicillins hereinbefore mentioned, e.g. benzyl penicillin or ampicillin, and including 6-APA itself and penicillanic acid. Other water-labile haptens applicable in the present invention are cephuroxime, cephalexin and other cephalosporins.

As the protein component, there may be used any suitable protein capable of coupling or conjugating with the hapten to form the desired immunogen. Sulphonated proteins are preferred for ease of cross-linking to the hapten by reaction with MBS(I) and MBS(II). Experiments so far carried out have been based on ovalbumin and sulphonated ovalbumin, and satisfactory results have been obtained. However, equally satisfactory results are predictable for other proteins.

Utilizing water-insoluble immunogens produced as above, antibodies may be raised to the hapten component in accordance with techniques well established in the art. For this purpose, the water-insoluble immunogen will be injected into a host animal, e.g. sheep or a mouse, to stimulate the production of antibodies in the host animal, followed either by recovery of the product antibodies from blood or blood serum drawn from the host animal, or, more usually and preferably, by monoclonal antibody-producing hybridoma technology. In that case, tissue cells from the immunised host animal having the ability to produce the required antibody, are recovered from the host animal, fused with myeloma cells to establish an immortal hybridoma cell line capable of producing the required antibody. Monoclonal antibody, e.g. the desired and preferred monoclonal antibody of this invention, i.e. specific to free, non-derivatised penicillin, can then be obtained by culturing the hybridoma cell line in vivo in a suitable host animal, or in vitro in a suitable culture medium, to accumulate the monoclonal antibody in the host animal, or the culture medium, and recovering the accumulated antibody. Such techniques are conventional and do not need to be described in detail.

In initially raising the desired antibodies, or antibody producing cells in the host animal, the water-insoluble immunogen may be injected in suspension in any suitable, injectable carrier medium, including aqueous media. However, non-aqueous carrier media are preferred.

The invention is illustrated by the following Examples.

## EXAMPLE 1 (Comparative)

### Preparation of penicillin immunogen in solution in aqueous media

10 mg of MBS(II), maleimidobutyryloxy-N-succinimide, in 1 ml tetrahydrofuran were added to 7.15 mg of 6-aminopenicillanic acid in 0.2 ml water, and the mixture stirred for 15 minutes. The mixture was then evaporated to dryness and the residue washed with 2:1 (vol. ratio) diethylether/dichloromethane. The solvent was removed to waste and to the residue was added 5 mg sulphonated ovalbumin in 1 ml tetrahydrofuran/water (1:1 vol. ratio) and mixed for 5 minutes. The protein conjugate was then immediately passaged through an ion-exchange column (Sephadex G-25) for a maximum of 5 minutes and mixed with Freund's adjuvant in a 1:1 vol. ratio to give a final protein concentration of 1 mg/ml.

## EXAMPLE 2 (Comparative)

### Preparation of water-insoluble penicillin immunogens using non-aqueous solvents

10 mg quantities of MBS(I) (maleimidobenzoyl-N-succinimide) were added to each of 7.15 mg 6-APA and 11.55 mg ampicillin each suspended in 5 ml tetrahydrofuran and agitated for 90 minutes. After evaporation to dryness, unreacted MBS(1) was removed by extraction with a diethylether-dichloromethane mixture (2:1 vol. ratio).

Following solvent extraction, the residues were resuspended each in 5 ml fresh tetrahydrofuran and 5 mg solid sulphonated ovalbumin added slowly and the mixtures stirred for 1 hour. The product protein conjugates were centrifuged off, washed with tetrahydrofuran and dried.

## EXAMPLE 3

Preparation of water-insoluble penicillin immunogens (precipitation from aqueous media)

7.2 µl tributylamine were added to 10 mg benzyl penicillin suspended in 3 ml dioxan and the mixture cooled in ice. 11.6 µl isobutyl chloroformate were added and the mixture stirred for 30 minutes at 4°C. The mixture was then added rapidly with stirring to an ice cold solution of 12.5 mg ovalbumin in 5 ml water. Precipitation occured within 10 minutes and the precipitate recovered by centrifuging to separate the supernatant, and washing twice with dioxan. The basic method is that disclosed by Erlanger B.F. et al (1959) J. Biol. Chem. 1090-1094, but modified in that excess chloroformate is used to precipitate the protein-hapten conjugate.

## EXAMPLE 4

Antibody production

Ovalbumin-penicillin conjugates (immunogens) prepared as in Examples 1, 2 and 3 were suspended in Freund's adjuvant and homogenised at high speed to give final suspensions having a protein concentration of 1 mg/ml. Freshly prepared suspensions (1 ml) were then administered intradermally and intramuscularly into sheep at 5 week intervals. A final booster injection of 2 mg immunogen emulsified in 10 ml sterile saline was given intravenously.

Blood samples from the immunised sheep were recovered shortly (between 3 and 13 days) after each boost, and antisera obtained therefrom assessed for titre and affinity ratio relative to benzyl penicillin and penicilloic acid, using standard radioimmunoassay (RIA) procedures.

The results obtained were as follows:

| Immunogen | Boost No. | Date of Immunisation or Boost | Titre* | Date of Bleed | Affinity Ratio** |
|---|---|---|---|---|---|
| 6-APA/ovalbumin conjugate (Example 1, comparative) | 1 | 13.2.87 | 1/20 | 20.2.87 | 1:1 |
| 6-APA/ovalbumin conjugate (Example 1, comparative) | 2 | 10.3.87 | 1/80 | 20.3.87 | 1:3 |
| 6-APA/ovalbumin conjugate (Example 1, comparative) | 3 | 5.6.87 | 1/320 | 20.6.87 | 1:10 |
| 6-APA/ovalbumin conjugate (Example 2, comparative) | 1 | 13.2.87 | 1/40 | 19.2.87 | 2:1 |
| 6-APA/ovalbumin conjugate (Example 2, comparative) | 2 | 12.3.87 | 1/50 | 16.3.87 | 3:1 |
| ampicillin/ovalbumin conjugate (Example 2, comparative) | 1 | 20.2.87 | 1/10 | 4.3.87 | 4:1 |
| ampicillin/ovalbumin conjugate (Example 2, comparative) | 2 | 29.4.87 | 1/320 | 9.4.87 | 9:1 |
| benzylpenicillin/ovalbumin conjugate (Example 3) | 1 | 6.2.87 | 1/640 | 18.2.87 | 1:1 |
| benzylpenicillin/ovalbumin conjugate (Example 3) | 2 | 10.3.87 | 1/64,00 | 19.3.87 | 9:1 |
| benzylpenicillin/ovalbumin conjugate (Example 3) | 3 | 8.5.87 | 1/16,000 | 12.5.87 | 6:1 |
| benzylpenicillin/ovalbumin conjugate (Example 3) | 4 | 16.7.87 | 1/50,000 | 29.7.87 | 100:1 |
| benzylpenicillin/ovalbumin conjugate (Example 3) | 5 | 13.7.88 | 1/100,000 | 22.7.88 | 50:1 |

* Titre: minimum dilution required to bind 50% by wt. of the free hapten, viz. benzyl penicillin.
** Affinity ratio: ratio of bound hapten, viz. benzyl penicillin to bound penicilloic acid.

The results presented demonstrate the high titre (1/50,000) and high affinity (100:1) to free, non-derivatised penicillins of antibodies raised using immunogenic protein conjugates of water-labile haptens produced in accordance with this invention (Example 3), that is to say by the substantially simultaneous precipitation of the protein/hapten conjugate from solution before any substantial degradation of the water-labile hapten occurs, as opposed to methods where no such precipitaton takes place (Example 1), or where the protein/hapten conjugate is prepared wholly in non-aqueous media (Example 2).

## EXAMPLE 5

### Immunoassay of penicillin in milk

Using standard enzyme labelled immunoassay (ELISA) and photometry procedures, anti-penicillin antibodies raised as in Example 4 and using the benzylpenicillin/ovalbumin conjugate of Example 3 were used in an ELISA assay of benzylpenicillin in cow's milk, both whole milk and at 50% dilution with water, and to which benzylpenicillin had been added in known amount. Two assays were performed, one with a 5 minute incubation period, and the other 30 minutes, both using antibody at a dilution of 1/1,000. The results are presented below in tabular form, Tables 1 to 3, and in graphical form in Figures 1 to 3 of the accompanying drawings. The measured absorbancies are the average of four separate milk samples.

TABLE 1

Whole Milk Samples (5 minute incubation)

| Added benzyl penicillin concentration, ng/ml | Measured absorbance | % of colour remaining |
|---|---|---|
| 3.125 | 0.391 | 65 |
| 6.25 | 0.319 | 53 |
| 12.5 | 0.289 | 48 |
| 25 | 0.252 | 42 |
| 50 | 0.214 | 36 |
| 100 | 0.171 | 29 |
| 200 | 0.131 | 22 |
| 400 | 0.102 | 17 |

TABLE 2

50% Aqueous Milk Samples (5 minute incubation)

| Benzyl penicillin concentration, ng/ml | Measured absorbance | % of colour remaining |
|---|---|---|
| 3.125 | 0.520 | 68 |
| 6.25 | 0.434 | 57 |
| 12.5 | 0.363 | 48 |
| 25 | 0.331 | 44 |
| 50 | 0.268 | 35 |
| 100 | 0.226 | 30 |
| 200 | 0.177 | 23 |
| 400 | 0.143 | 19 |

TABLE 3

Whole Milk Samples (30 minute incubation)

| Benzyl penicillin concentration, ng/ml | Measured absorbance | % of colour remaining |
|---|---|---|
| 3.125 | 0.903 | 74 |
| 6.25 | 0.721 | 59 |
| 12.5 | 0.635 | 52 |
| 25 | 0.532 | 44 |
| 50 | 0.437 | 36 |
| 100 | 0.362 | 30 |
| 200 | 0.269 | 22 |
| 400 | 0.217 | 18 |

EXAMPLE 6

Preparation of water-insoluble cepholosporin immunogens

Following the same general procedure as Example 3, that is to say by cross-linking in the presence of excess cross-linking agent, and using the same relative molar proportions of the reactants and either cephuroxime or cephalexin, i.e. with amounts of cephuroxime and cephalexin adjusted relative to the reactants to reflect the different molecular weights of cephuroxime and cephalexin relative to benzylpenicillin, water-insoluble cephalosporin immunogens were prepared, viz: a water-insoluble cephuroxime/ovalbumin conjugate and a water-insoluble cephalexin/ ovalbumin conjugate.

Preparation of anti-cephalosporin antibodies

Water-insoluble cephalosporin immunogens prepared as above were suspended in Freund's adjuvant and homogenised at high speed to provide final suspensions having a protein concentration of 1 mg/ml. 1 ml quantities of the freshly prepared suspensions were administered intradermally and intramuscularly to sheep at 5 week intervals followed by a final booster intravenous injection of 2 mg of immunogen emulsified in 10 ml sterile saline. The following immunisation protocol was followed: initial immunisation 11th September, 1987; boosts 17th December, 1987, 1st February, 1988, 8th March, 1988, 18th May, 1988.

Blood samples from the immunised sheep were recovered at intervals following each administration and antisera separated therefrom were assessed for specific binding to free, non-derivatised hapten (cephuroxime or cephalexin) in cow's milk using enzyme-labelled cephuroxime and cephalexin in a conventional competitive ELISA procedure. The results for the anti-cephuroxime antibody are presented below, Tables 5 and 6. Similar results were obtained using the anti-cephalexin antisera. All optical density (OD) are corrected for background and are the average of four measurements.

TABLE 5

Optical Densities, Whole Milk Sample Containing 10 ng cephuroxime, 15 minute incubation

| Date of Bleed | Antibody Concentration | Average OD Negative Control | Average OD Sample | % Colour Retained |
|---|---|---|---|---|
| 28.03.88 | 1/400 | 0.613 | 0.233 | 38 |
| | 1/800 | 0.508 | 0.205 | 40 |
| | 1/1600 | 0.436 | 0.174 | 40 |
| | 1/3200 | 0.377 | 0.167 | 44 |
| 02.06.88 | 1/400 | 0.616 | 0.239 | 39 |
| | 1/800 | 0.505 | 0.178 | 35 |
| | 1/1600 | 0.424 | 0.155 | 37 |
| | 1/3200 | 0.333 | 0.123 | 37 |
| 06.06.88 | 1/400 | 0.523 | 0.205 | 39 |
| | 1/800 | 0.508 | 0.190 | 37 |
| | 1/1600 | 0.495 | 0.189 | 38 |
| | 1/3200 | 0.444 | 0.161 | 36 |

TABLE 6

Optical Densities, Whole Milk Samples 15 minute incubation

| Cephuroxime Concentration (ng/ml) | Average OD Negative Control | Average OD Sample | % Colour Retained |
|---|---|---|---|
| 1.563 | 0.262 | 0.216 | 82 |
| 3.125 | 0.249 | 0.183 | 74 |
| 6.25 | 0.237 | 0.140 | 59 |
| 12.5 | 0.270 | 0.119 | 44 |
| 25 | 0.252 | 0.089 | 36 |
| 50 | 0.249 | 0.085 | 34 |

The results tabulated in Table 6 are presented graphically in Figure 4 of the accompanying drawings.

## EXAMPLE 7

Using standard ELISA techniques, cross-reactivities of the anti- penicillin antibody raised using the water-insoluble immunogenic benzyl penicillin/ovalbumin conjugate of Example 3 were determined and are tabulated below, Table 7.

## TABLE 7

## Cross reactivities, benzyl penicillin (Pen.G) antibody (Example 3)

| Cross-Reactant | Cross-Reactant 6 ng/ml | 60 ng/ml | 600 ng/ml | Control OD | % Colour Remaining(1) 6 ng/ml | 60 ng/ml | 600 ng/ml | Pen.G (6 ng/ml) OD | Antibody % Colour Remaining(2) | Calculated ng/ml(3) | % Cross Reactivity |
|---|---|---|---|---|---|---|---|---|---|---|---|
| Cephuroxime | 0.311 | 0.300 | 0.283 | 0.309 | 101 | 99 | 92 | 0.190 | 62 | - | <0.5 |
| 6-APA | 0.323 | 0.312 | 0.320 | 0.329 | 98 | 95 | 97 | 0.220 | 68 | - | <0.5 |
| Oxacillin | 0.325 | 0.318 | 0.285 | 0.339 | 96 | 94 | 84 | 0.212 | 63 | - | <0.5 |
| Cephalexin | 0.323 | 0.324 | 0.306 | 0.342 | 94 | 95 | 90 | 0.216 | 63 | - | <0.5. |
| Tetracyline | 0.315 | 0.176 | 0.203 | 0.278 | 113 | 63 | 73 | 0.155 | 56 | - | <0.5 |
| Penicillinamine acetone | 0.215 | 0.209 | 0.180 | 0.265 | 81 | 79 | 68 | 0.176 | 66 | 760 | 0.8 |
| Penicillinamine disulphide | 0.196 | 0.204 | 0.163 | 0.280 | 70 | 73 | 58 | 0.172 | 61 | 430 | 1.4 |
| Hippuric acid | 0.208 | 0.206 | 0.203 | 0.285 | 73 | 72 | 71 | 0.172 | 60 | - | <0.5 |
| Phenethicillin | 0.216 | 0.217 | 0.158 | 0.298 | 73 | 73 | 53 | 0.200 | 67 | 170 | 3.5 |
| Phenoxymethyl-penicillanic acid benzyl ester | 0.180 | 0.177 | 0.148 | 0.230 | 78 | 77 | 64 | 0.158 | 69 | 300 | 2.0 |
| Methicillin | 0.192 | 0.187 | 0.179 | 0.249 | 77 | 75 | 72 | 0.166 | 67 | - | <0.5 |
| Sulphaquinoxaline | 0.188 | 0.191 | 0.178 | 0.245 | 77 | 78 | 73 | 0.174 | 71 | - | <0.5 |
| Sulphamethazine | 0.185 | 0.191 | 0.180 | 0.244 | 76 | 78 | 74 | 0.163 | 67 | - | <0.5 |
| 7-aminocephalo-sporanic acid | 0.200 | 0.196 | 0.184 | 0.247 | 81 | 79 | 77 | 0.170 | 69 | - | <0.5 |
| Penicilloic acid | 0.162 | 0.166 | 0.114 | 0.234 | 69 | 71 | 49 | 0.160 | 68 | 160 | 3.8 |
| Penicillamine | 0.190 | 0.187 | 0.175 | 0.227 | 84 | 82 | 77 | 0.169 | 75 | 720 | 0.8 |
| Cephalonium | 0.204 | 0.176 | 0.113 | 0.276 | 74 | 64 | 41 | 0.172 | 62 | 80 | 7.5 |
| Oxytetracycline | 0.310 | 0.285 | 0.276 | 0.297 | 104 | 96 | 93 | 0.177 | 60 | - | <0.5 |
| Cephalosporin C | 0.305 | 0.326 | 0.306 | 0.349 | 87 | 93 | 88 | 0.215 | 62 | - | <0.5 |
| Cloxacillin | 0.347 | 0.351 | 0.323 | 0.366 | 95 | 96 | 83 | 0.227 | 62 | - | <0.5 |
| Nafcillin | 0.351 | 0.359 | 0.339 | 0.375 | 94 | 96 | 90 | 0.247 | 66 | - | <0.5 |
| Chloramphenicol | 0.382 | 0.363 | 0.370 | 0.389 | 98 | 93 | 95 | 0.247 | 64 | - | <0.5 |
| Cephalothin | 0.359 | 0.283 | 0.177 | 0.394 | 91 | 72 | 45 | 0.258 | 66 | 105 | 5.7 |
| Streptomycin | 0.401 | 0.412 | 0.374 | 0.429 | 94 | 96 | 87 | 0.273 | 64 | - | <0.5 |
| Ampicillin | 0.386 | 0.387 | 0.269 | 0.430 | 90 | 90 | 63 | 0.262 | 61 | 640 | 0.9 |
| Penicillin V | 0.377 | 0.285 | 0.122 | 0.415 | 91 | 69 | 29 | 0.267 | 64 | 90 | 6.7 |
| Cephadrine | 0.279 | 0.275 | 0.259 | 0.286 | 98 | 96 | 91 | 0.161 | 56 | - | <0.5 |

(1) % colour remaining - $\left(\frac{\text{cross reactant OD}}{\text{control OD}}\right) \times 100$

(2) % colour remaining - $\left(\frac{\text{Pen.G antibody OD}}{\text{control OD}}\right) \times 100$

(3) Calculated ng/ml - calculated to give same % difference as 6 ng/ml Pen.G.

EXAMPLE 8

A chequerboard ELISA titration for cephalexin antibody (Example 6) versus cephalexin enzyme conjugate was carried out using standard ELISA chequerboard techniques. The cephalexin antibody sera were obtained from blood samples recovered from the immunised animal (sheep) on 6th January, 1988 and 9th February, 1988 following the injection protocol given in Example 6. The absorbance values (OD, corrected for background) are presented in Table 8. Titrations using sera recovered on 6th January, 1988 were performed using a one hour incubation period; those on 9th February, 1988 were titrated following a half hour incubation.

TABLE 8

| Conjugate Concentrations | Date of Bleed | Sera Dilutions | | | | | |
|---|---|---|---|---|---|---|---|
| | | 1/100 | 1/200 | 1/400 | 1/800 | 1/1600 | 1/3200 |
| 1/50 | 6.1.88 | - | - | - | - | - | - |
| | 9.2.88 | 0.194 | - | <0.4 | 0.205 | - | - |
| 1/100 | 6.1.88 | 0.640 | - | - | - | - | - |
| | 9.2.88 | 1.693 | 1.652 | >1.6 | >1.6 | 1.334 | 0.833 |
| 1/200 | 6.1.88 | 1.445 | 1.252 | - | - | - | - |
| | 9.2.88 | 1.598 | 1.665 | >1.8 | 1.707 | 1.415 | 0.760 |
| 1/400 | 6.1.88 | >1.8 | >1.741 | >1.667 | >1.404 | 1.152 | 0.785 |
| | 9.2.88 | 1.814 | 1.840 | 1.791 | 1.590 | 1.299 | 0.707 |
| 1/800 | 6.1.88 | >1.988 | >1.947 | 1.689 | 1.253 | 1.054 | 0.729 |
| | 9.2.88 | 1.873 | 1.852 | 1.715 | 1.491 | 1.122 | 0.625 |
| 1/1600 | 6.1.88 | 1.754 | 1.596 | 1.365 | 0.986 | 0.868 | 0.588 |
| | 9.2.88 | 1.618 | 1.620 | 1.493 | 1.191 | 0.877 | 0.481 |
| 1/3200 | 6.1.88 | 0.985 | 1.075 | 0.869 | 0.690 | 0.556 | 0.423 |
| | 9.2.88 | 1.392 | 1.346 | 1.130 | 0.869 | 0.671 | 0.364 |
| 1/6400 | 6.1.88 | 0.794 | 0.640 | 0.567 | 0.449 | 0.344 | 0.251 |
| | 9.2.88 | 1.007 | 1.059 | 0.859 | 0.680 | 0.451 | 0.235 |

## Claims

1. A method of producing an antigenic protein-hapten conjugate from a water-labile hapten, which comprises forming the protein-hapten conjugate in an aqueous medium, and recovering the conjugate in water-insoluble form from the aqueous medium before any substantial degree of degradation has taken place of the water-labile hapten.

2. A method according to claim 1, wherein the protein-hapten conjugate is initially formed in solution in said aqueous medium, and is precipitated from said medium in water-insoluble form before any substantial degree of degradation has taken place of the water-labile hapten.

3. A method according to claim 2, wherein the protein-hapten conjugate is precipitated from solution by subsequent treatment of protein conjugate solution after formation of the protein conjugate, but before any substantial degree of degradation of the water-labile hapten.

4. A method according to claim 2, wherein the protein-hapten conjugate is precipitated from solution by adjustment of the pH of the solution or salting out.

5. A method according to claim 2, wherein the formation and precipitation of the protein-hapten conjugate in and from the solution, respectively, take place substantially simultaneously.

6. A method according to claim 5, wherein the protein-hapten conjugate is formed in solution by reaction of the protein and the hapten in the presence of a cross-linking agent, and wherein the cross-linking agent is used not only in an amount sufficient to effect the coupling of the hapten to the protein, but also in an additional amount sufficient to effect the substantially simultaneous cross-linking and insolubilisation of the protein, and the consequent substantially simultaneous precipitation of the protein-hapten conjugate.

7. A method according to claim 5, wherein the cross-linking agent used is maleimidobenzoyl-N-succinimide, maleimidobutyryloxy-N-succinimide, dimethyladipimidate, carbodiimide or isobutylchloroformate.

8. A method according to claim 1, wherein the protein is insolubilised prior to conjugation with the hapten, and the hapten subsequently conjugated to the insoluble protein to provide a water-insoluble protein-hapten conjugate.

9. A method according to any one of claims 1-8, wherein the water-labile hapten is a penicillin or cephalosporin.

10. A method according to claim 9, wherein the water-labile hapten is penicillanic acid, 6-aminopenicillanic acid, benzyl penicillin, ampicillin, cephuroxime or cephalexin.

11. A method according to any one of claims 1-10, wherein the protein is a sulphonated protein.

12. A method according to claim 11, wherein the protein is a sulphonated ovalbumin.

13. A method of raising antibodies in a host animal, which antibodies are specific to a water-labile hapten, which comprises injecting into the host animal a protein-hapten conjugate capable of stimulating the production of said antibodies by the host animal, and recovering the antibodies, or animal tissue cells capable of producing said antibodies, from the host animal, wherein the said protein-hapten conjugate is used in water-insoluble form.

14. A method according to claim 13, wherein the water-insoluble protein-hapten conjugate is injected into the host animal in suspension in a non-aqueous injectable carrier.

15. A method according to claim 13 or 14, wherein the protein-hapten conjugate used is a water-insoluble protein-hapten conjugate obtained by a method claimed in any one of claims 1-12.

16. A method according to claim 13, 14 or 15, wherein, following injection of the water-insoluble protein-hapten conjugate into the host animal, antibody producing cells capable of producing antibodies specific to said hapten are recovered from said host animal, and fused with a myeloma cell line, thereby to establish a perpetual hybridoma cell line having the ability to produce said antibody in monoclonal form.

17. A method according to claim 16, including the additional step or steps of culturing the hybridoma cell line in vivo or in vitro thereby to stimulate the production and accumulation of monoclonal antibody, and recovering the accumulated monoclonal antibody.

18. An antigenic, water-insoluble protein-hapten conjugate when prepared by a method claimed in any one of claims 1-12.

19. An antigenic, water-insoluble protein-hapten conjugate according to claim 18, wherein the hapten component is a penicillin or cephalosporin.

20. An antigenic, water-insoluble protein-hapten conjugate according to claim 19, wherein the hapten component is penicillanic acid, 6-aminopenicillanic acid, benzyl penicillin, ampicillin, cephuroxime or cephalexin.

21. An antibody having a specificity towards a water-labile hapten, when prepared by a method claimed in any one of claims 13 to 17.

22. An antibody according to claim 21, which is specific to a free, non-derivatised penicillin or cephalosporin.

23. An antibody according to claim 22, which is specific to free, non-derivatised penicillanic acid, 6-aminopenicillanic acid, benzyl penicillin, ampicillin, cephuroxime or cephalexin.

24. An immunoassay procedure for the determination of water-labile haptens present in a sample, wherein there is used an antibody as claimed in claim 21.

25. An immunoassay procedure according to claim 24, as applied to the determination of beta-lactam antibiotics present in a sample.

26. An immunoassay procedure according to claim 25, as applied to the determination of penicillin in a sample.

27. An immunoassay procedure according to any one of claims 24 to 26, wherein the sample is a milk sample.

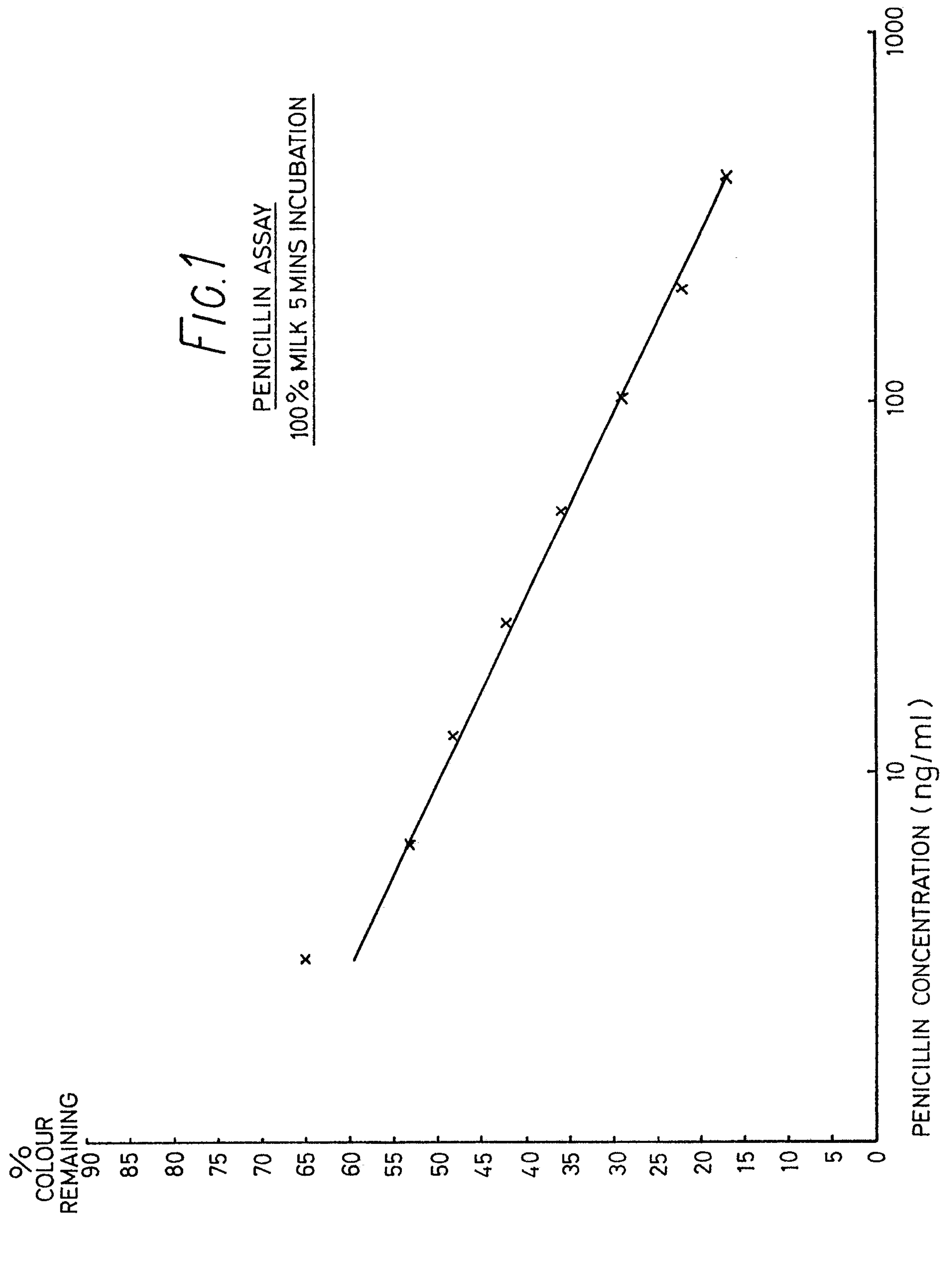
PENICILLIN ASSAY
100% MILK 5 MINS INCUBATION
% COLOUR REMAINING
90
85
80
75
70
65
60
55
50
45
40
35
30
25
20
15
10
5
0
10
100
1000
PENICILLIN CONCENTRATION ( ng/ml )

FIG.1

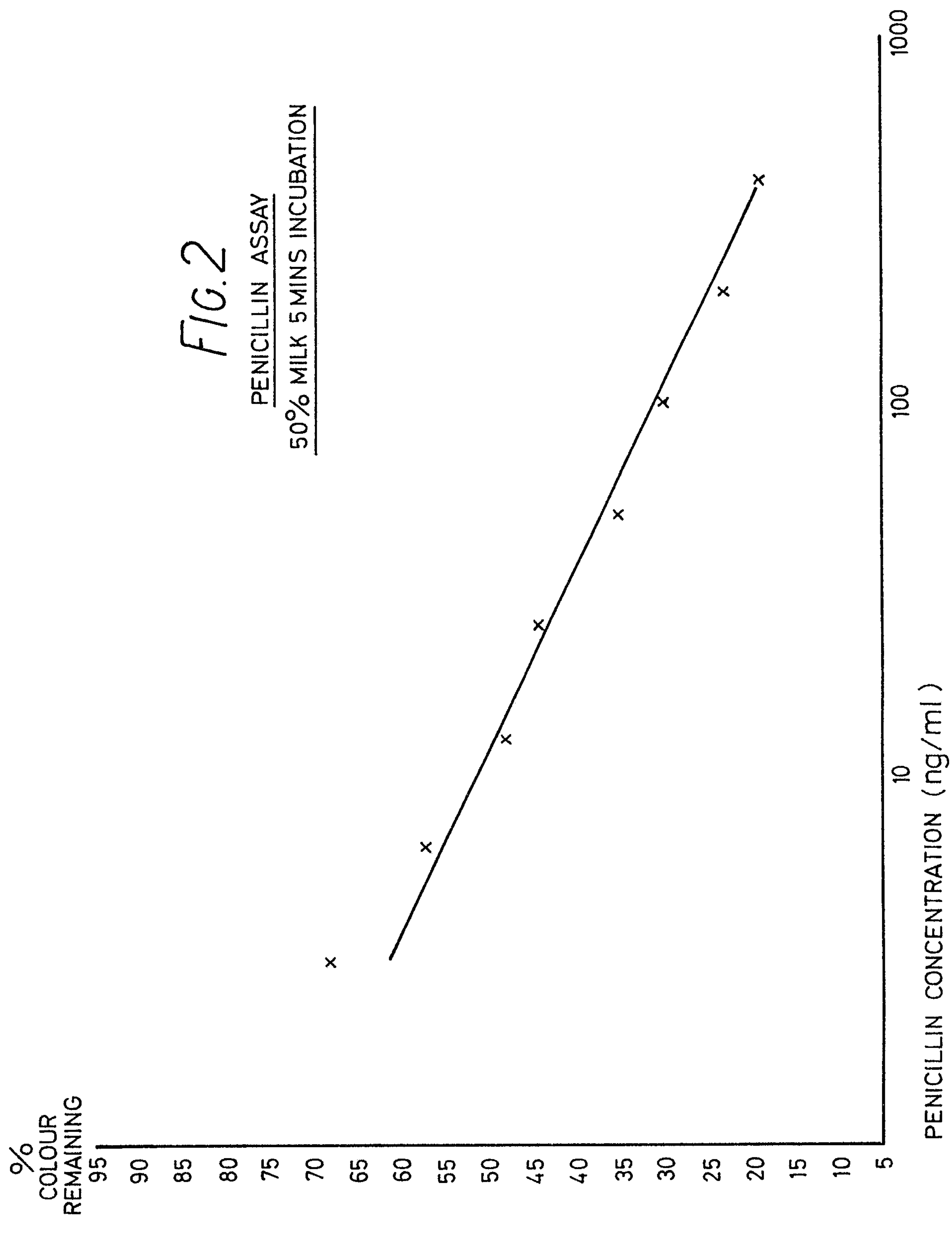
PENICILLIN ASSAY
50% MILK 5 MINS INCUBATION
% COLOUR REMAINING
95
90
85
80
75
70
65
60
55
50
45
40
35
30
25
20
15
10
5
10
100
1000
PENICILLIN CONCENTRATION (ng/ml)

FIG. 2

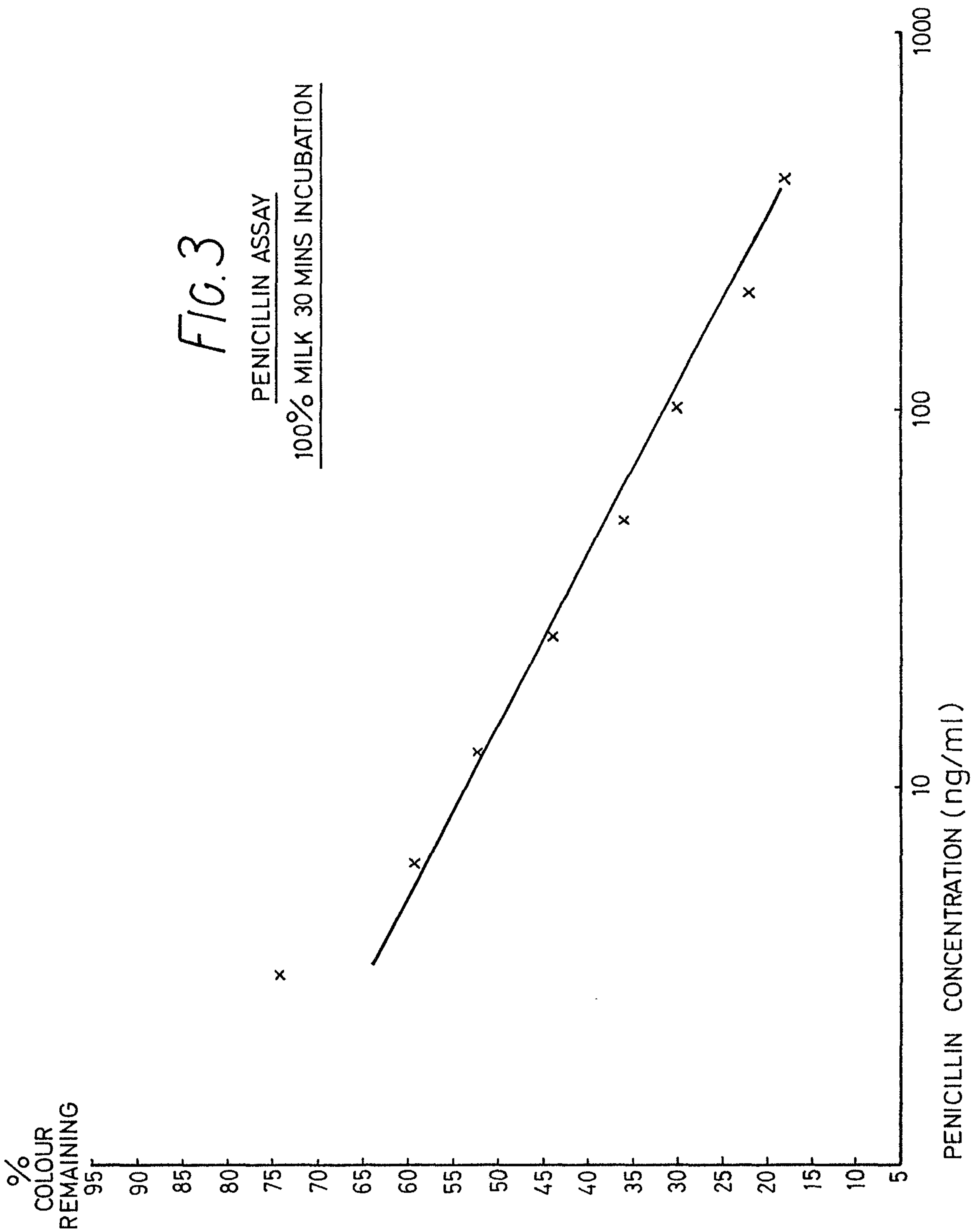


FIG. 3

PENICILLIN ASSAY

100% MILK 30 MINS INCUBATION

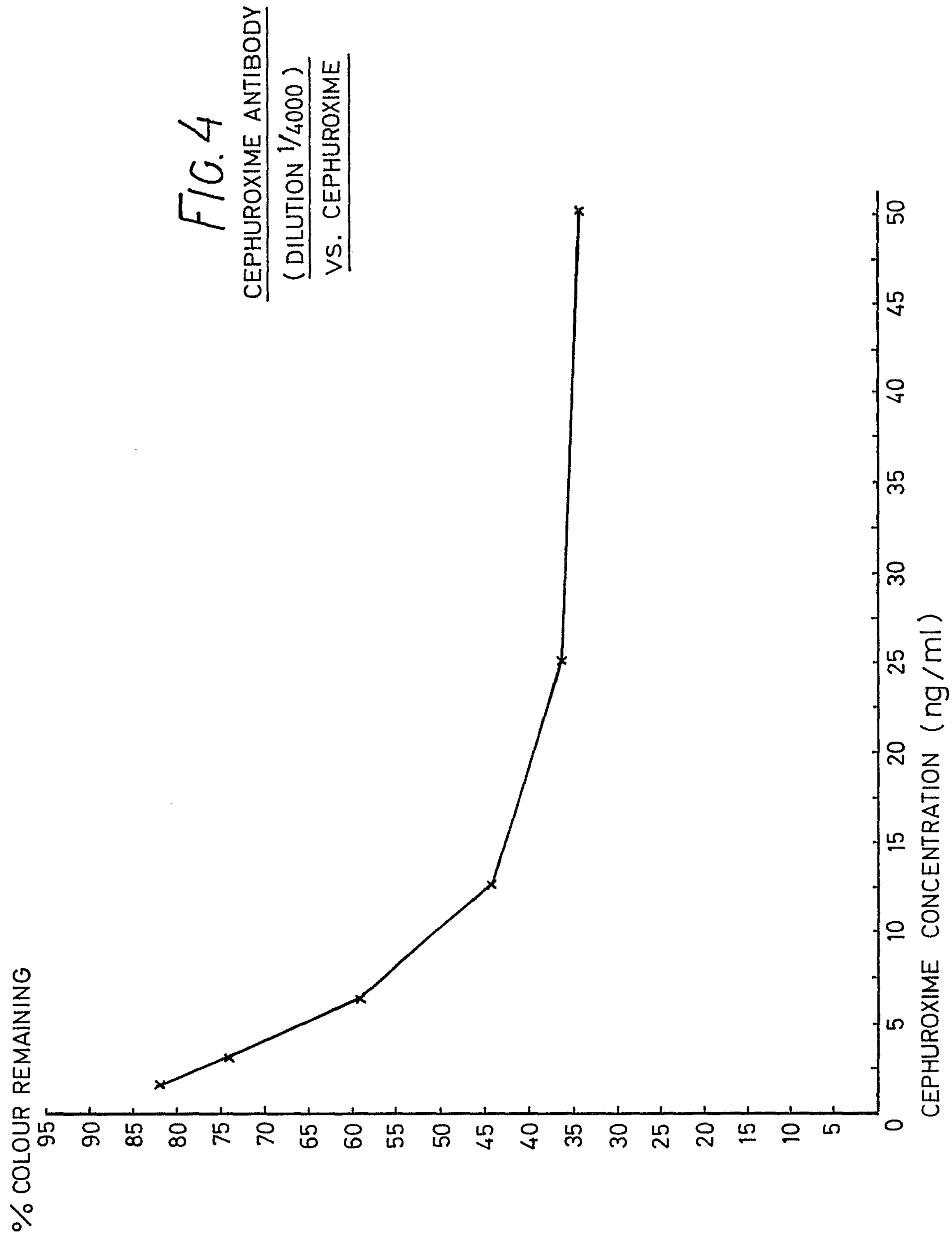
CEPHUROXIME ANTIBODY
( DILUTION 1/4000 )
VS. CEPHUROXIME
% COLOUR REMAINING
95 90 85 80 75 70 65 60 55 50 45 40 35 30 25 20 15 10 5 0
0 5 10 15 20 25 30 35 40 45 50
CEPHUROXIME CONCENTRATION ( ng/ml )

FIG. 4

European Patent Office

# EUROPEAN SEARCH REPORT

Application Number

EP 88 30 8919

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X<br><br>Y | US-A-4 252 784 (B.B.LEVINE)<br>* Column 1, line 1 - column 4, line 48 * | 1-4,9, 10,18-20<br>13-17, 21-27 | G 01 N 33/531<br>C 07 K 15/00<br>G 01 N 33/94<br>C 12 P 21/00 |
| Y,D | EP-A-0 144 154 (SYNTEX (USA) INC.)<br>* The whole document * | 13-17, 21-27 | |
| Y | US-A-4 096 138 (G.H.SCHERR)<br>* Column 1, line 1 - column 7, line 4 * | 1,6-10, 13,15, 18-27 | |
| Y,D | WO-A-8 603 841 (INTERNATIONAL IMMUNOASSAY LABS. INC.)<br>* The whole document * | 1,6-10, 13,15, 18-27 | |
| A | GB-A-2 004 744 (A.R.SANTERSON)<br>* Page 2, lines 5-51; page 3, example 2 * | 7,11,12 | |

TECHNICAL FIELDS SEARCHED (Int. Cl.4)

G 01 N
A 61 K

*The present search report has been drawn up for all claims*

| Place of search | Date of completion of the search | Examiner |
|---|---|---|
| THE HAGUE | 06-12-1988 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)